# EUROPEAN PATENT SPECIFICATION

(11) **EP 1 771 551 B1**
(45) Date of publication and mention of the grant of the patent: **05.01.2011**
(21) Application number: 05776601.6
(22) Date of filing: 20.07.2005
(51) Int. Cl.: C12N 5/0789, C12N 5/074, C12N 5/071, A61K 45/00

(54) **NOVEL CELL POPULATIONS AND USES THEREOF**
NEUE ZELLPOPULATIONEN UND DEREN ANWENDUNGEN
NOUVELLES POPULATIONS CELLULAIRES ET UTILISATIONS DE CELLES-CI

(30) Priority: 20.07.2004 US 589941 P
(43) Date of publication of application: 11.04.2007
(73) Proprietor: CASE WESTERN RESERVE UNIVERSITY, Cleveland, OH 44106-7219 (US)
(72) Inventor: LAUGHLIN, Mary, J., Shaker Heights, OH 44122 (US); POMPILI, Vincent, Hudson, OH 44236 (US)
(74) Representative: Vossius & Partner
(86) International application number: PCT/US2005/025854
(87) International publication number: WO 2006/012404

(56) References cited:
- WALENTA K ET AL: "In vitro differentiation characteristics of cultured human mononuclear cells-implications for endothelial progenitor cell biology" BIOCHEMICAL AND BIOPHYSICAL RESEARCH COMMUNICATIONS, ACADEMIC PRESS INC. ORLANDO, FL, US, vol. 333, no. 2, 29 July 2005 (2005-07-29), pages 476-482, XP004949508 ISSN: 0006-291X
- GREGORY C A ET AL: "Non-hematopoietic bone marrow stem cells: Molecular control of expansion and differentiation" EXPERIMENTAL CELL RESEARCH, SAN DIEGO, CA, US, vol. 306, no. 2, 10 June 2005 (2005-06-10), pages 330-335, XP004909297 ISSN: 0014-4827
- BONANNO GIUSEPPINA ET AL: "Clinical isolation and functional characterization of cord blood CD133+ hematopoietic progenitor cells" TRANSFUSION (MALDEN), vol. 44, no. 7, July 2004 (2004-07), pages 1087-1097, XP002349736 ISSN: 0041-1132
- KUWANA M ET AL: "Defective vasculogenesis in systemic sclerosis" LANCET THE, LANCET LIMITED. LONDON, GB, vol. 364, no. 9434, 14 August 2004 (2004-08-14), pages 603-610, XP004746776 ISSN: 0140-6736
- SCHMEISSER A ET AL: "Monocytes coexpress endothelial and macrophagocytic lineage markers and form cord-like structures in Matrigel under angiogenic conditions" CARDIOVASCULAR RESEARCH, vol. 49, no. 3, 2001, pages 671-680, XP002979381 ISSN: 0008-6363
- LODIE T A ET AL: "Systematic analysis of reportedly distinct populations of multipotent bone marrow-derived stem cells reveals a lack of distinction" TISSUE ENGINEERING, LARCHMONT, NY, US, vol. 8, no. 5, October 2002 (2002-10), pages 739-751, XP002306489 ISSN: 1076-3279
- FINNEY MARCIE ET AL: "Umbilical cord blood versus adult bone marrow-derived endothelial precursor cells (EPCs): In vitro characteristics and effectiveness in vivo in mediating neovascularization after femoral artery injury." CIRCULATION, vol. 108, no. 17 Supplement, 28 October 2003 (2003-10-28), pages IV-111, XP009055361 & AMERICAN HEART ASSOCIATION SCIENTIFIC SESSIONS 2003; ORLANDO, FL, USA; NOVEMBER 09-12, 2003 ISSN: 0009-7322
- FINNEY M ET AL: "Comparison of Umbilical Cord Blood Versus Bone Marrow-Derived Endothelial Precursor Cells in Mediating Neovascularization in a NOD/SCID Hind Limb Injury Model." BLOOD, vol. 100, no. 11, 16 November 2002 (2002-11-16), page Abstract No. 2691, XP009055366 & 44TH ANNUAL MEETING OF THE AMERICAN SOCIETY OF HEMATOLOGY; PHILADELPHIA, PA, USA; DECEMBER 06-10, 2002 ISSN: 0006-4971
- KALKA CHRISTOPH ET AL: "Transplantation of ex vivo expanded endothelial progenitor cells for therapeutic neovascularization" PROCEEDINGS OF THE NATIONAL ACADEMY OF SCIENCES OF USA, NATIONAL ACADEMY OF SCIENCE, WASHINGTON, DC, US, vol. 97, no. 7, 28 March 2000 (2000-03-28), pages 3422-3427, XP002204951 ISSN: 0027-8424

## Description

### Related Applications

This application claims the benefit of the filing date of U.S. Provisional Application No. 60/589941, filed July 20,2004, entitled "NOVEL CD133- CELL POPULATIONS AND USES THEREOF." The entire teachings of the referenced application are incorporated by reference herein.

### Background of the Invention

Atherosclerotic cardiovascular disease is a leading cause of morbidity and mortality in the industrialized western hemisphere. Coronary artery disease, the pathologic process of arterial luminal narrowing by atherosclerotic plaque resulting in obstruction of blood flow to the heart, accounts for about half of the deaths. Although catheter-based revascularization or surgery-based treatment approaches have been successful in restoring blood flow to ischemic myocardium in the majority of cases, the treatments are inadequate for a significant number of patients who remain incompletely revascularized. The ramifications of treatment limitations may be significant in patients who have large areas of ischemic, but viable myocardium jeopardized by the impaired perfusion supplied by vessels that are poor targets for conventional revascularization techniques. Treatment alternatives, including mechanical approaches such as percutaneous transluminal myocardial revascularization, and the like, have not produced encouraging results. Gene therapy using adenoviral vectors to augment cytokine production and, therefore, promote angiogenesis has shown promise, but this therapy has limitations and has not yet emerged as the optimal treatment for these patients. Therefore, therapeutic angiogenesis has attracted many researchers attempting to discover a way to circumvent the burden of chronic myocardial ischemia.

Atherosclerosis of the extremities is a leading cause of occlusive arterial disease of the extremities in patients over age 40. Peripheral vascular occlusive disease and its complications, including ulcers and even necrosis of the affected limb, is also common. Although percutaneous transluminal angioplasty and aorto-bifemoral bypass procedures are associated with acceptable morbidity and mortality risk and are usually initially successful, these interventions have not been shown to be effective long-term.

Therefore, there is still a need to develop treatment modalities for both myocardial ischemia and peripheral vascular disease that can promote vasculogenesis in the ischemic tissue. The present invention provides novel cell populations which may be used in cell-based treatments for inducing neovascularization.

### Summary of the Invention

The invention provides, in part, novel population of cells termed Endothelial Generating Cells (EGCs). The invention provides compositions comprising EGCs, and in some embodiments, additional cell populations such stem cells, CD133⁺ cells, endothelial precursor cells, endothelial cells and mesenchymal stem cells. Another aspect of the invention provides kits comprising EGCs.

Another aspect of the invention provides uses for the EGC cell populations. One aspect of the invention provides methods of inducing neovascularization in subjects in need thereof, such as in subjects afflicted with ischemia or vascular occlusion, by administering the novel cell population alone or in conjuction with other cells or noncellular agents.

Another aspect of the invention provides a method of providing cellular therapies for inducing neovascularization, and methods for the distribution of a the novel EGC populations.

The invention further provides agents for the manufacture of medicaments to treat any of the disorders described herein. Any methods disclosed herein for treating or preventing a disorder by administering an agent to a subject may be applied to the use of the agent in the manufacture of a medicament to treat that disorder. For example, in one specific embodiment, EGCs, optionally in combination with CD133⁺ cells, may be used in the manufacture of a medicament for the treatment of cardiac or renal ischemia.

### Brief Description of the Drawings

Figure 1 shows the surface phenotyping of unselected mononuclear cells (NINC) and EGCs.
Figure 2 shows photomicrographs of lower calf muscle of mice 28 days after isolation and ligation of the right femoral artery and injection of cytokines (A), or endothelial precursor cells (EPCs) derived from umbilical cord blood (B) or from bone marrow (C). Samples were stained for alkaline phosphatase by the indoxyl-tetrazolium method and used to assess capillary density.
Figure 3 shows the density of capillaries formed in the calf muscle of mice 28 days after the ligation of the right femoral artery and injection of cytokines or EPC. Results are from a representative experiment.
Figure 4 shows the limb blood flow perfusion ratios in mice having femoral artery ligation and treated with the indicated cell populations derived from bone marrow.
Figure 5 shows the limb blood flow perfusion ratios in mice having femoral artery ligation and treated with the indicated cell populations derived from umbilical cord blood.
Figures 6 shows a bar graph of migration AC133⁺ cells, EGCs, MNCs or combinations thereof in response to SDF-1.
Figure 7 shows photomicrographs of migrating AC133⁺ cells, EGCs, MNCs or combinations thereof in response to SDF-1.
Figure 8 shows photomicrographs of HUVEC tubule formation in the presence of AC133⁺ cells and EGCs.

### Detailed Description of the Invention

### I. Overview

The invention broadly relates to a novel population of cells, termed "EGCs" and to related compositions, kits, methods for their isolation, and methods of using the cells for therapeutic treatments. The invention further provides methods of providing cellular therapies and of distributing EGC compositions such as to healthcare professionals. EGCs are CD34⁻CD133⁻CD73⁻ cells preferably expressing one or more markers selected from CD14⁺, CD11b⁺, CXCR4⁺, preferably at least two, and more preferably all three of these markers i.e. the EGCs are CD34⁻CD133⁻CD73⁻CD14⁺CD11b⁺CXCR4⁺ cells. In yet another embodiment, the EGCs express one or more of the following surface markers: intercellular adhesion molecule-1 (ICAM-1), monocyte chemotactic protein-1 (MAC-1), lymphocyte function-associated antigen-1 (LFA-1), endoglin (CD105), CD29, CD55, CD44, or combinations thereof. In another embodiment, the EGCs are also CD45'.

One aspect of the invention provides a composition comprising a population of human cells, wherein at least 10% of the cells are CD34⁻, CD133⁻, and CD73⁻; and wherein further the cells express one or more of the surface markers: CD14, CD11b and CXCR4. In one embodiment, the cells express two or more of CD14, CD11b and CXCR4. In another embodiment, the cells express CD14, CD11b and CXCR4. In another embodiment, at least 20% of the human cells are CD34⁻, CD133⁻, CD73⁻, CD14⁺, CD11b⁺, CXCR4⁺ cells. In another embodiment, at least 50% of the human cells are CD34⁻, CD133⁻, CD73⁻, CD14⁺, CD11b⁺, CXCR4⁺ cells. In another embodiment, the cells are positive for KDR or CD105 or both.

In another embodiment, the compositions provided by the invention comprise a physiologically and/or pharmaceutically acceptable medium. In a related embodiment, the composition is formulated as an injectable composition. The cell populations of the present invention may be isolated from multiple sources, including umbilical cord blood, bone marrow or peripheral blood. In preferred embodiments, the EGCs of the present invention grow as adherent cells in fibronectin-coated substrates. In other embodiments, the EGCs are capable of differentiating into stromal cells.

Another aspect of the invention provides compositions comprising EGCs and a second cell population, such as but not limited to CD133⁺ cells. One aspect of the invention provides a composition comprising a population of human cells, wherein (a) at least 10% of the cells are CD34⁻CD133⁻CD73⁻ cells which further express one or more of the surface markers CD14, CD11b and CXCR4; and wherein (b) at least 10% of the cells are CD133⁺ cells. In a related embodiment, at least 25% of the cells are CD133⁺. In some embodiments, the CD133⁺ cells are CD133⁺CD34⁺ cells, while in other embodiments they are CD133⁺CD34⁻ cells. In some embodiments, at least 10%, 20%, 30%, 40%, 50%, 60%, 70%, 80%, 90%, 95%, 98%, 99% or 100% of the cells in the composition are either EGCs or Cd133⁺ cells.

In compositions comprising more than one cell population, the multiple cell populations may be isolated from the same or from different sources. In one embodiment, the composition comprises EGCs and CD133⁺ cells both isolated from umbilical cord blood, either from the same cord blood or from blood of different cords.

Another aspect of the invention provides a composition comprising a population of human cells, wherein (a) at least 10% of the cells are EGCs; and (b) at least 10% of the cells are mesenchymal stem cells.

In some embodiments, at least one cell in the composition is genetically modified. The genetic modification may allow the expression of a recombinant polypeptide or a double stranded RNA. In some embodiments, the polypeptide is an antibody, a growth factor, a cytokine or a chemokine. In some embodiments, the polypeptide promotes the localization of the cells to sites of ischemic tissue. In some embodiments, the compositions further comprise an agent that (i) promotes vascularization or angiogenesis; or (ii) reduces inflammation or coagulation.

Another aspect of the invention provides kits comprising any one of the compositions of EGCs. In some embodiments, the compositions in the kits are cryopreserved. In some embodiments, the kits comprise an excipient suitable for intracoronary injection. In preferred embodiments, the excipient is a nonpyrogenic excipient. In another embodiment, the kit further comprises at least one anticoagulating agent. In one embodiment, the anticoagulating agent is an inhibitor of GP IIb-IIIa, thrombin or a vitamin K-dependent coagulation factor. In a specific embodiment, the anticoagulating agent is selected from the group consisting of eptifibatide, argatroban, ximelagatran, warfarin and heparin

In another embodiment, the kit comprises at least one component of a closed sterile system suitable for intra-coronary injection, such as a needle, syringe, catheter based syringe, needle-based injection device or needleless injection device.

Another aspect of the invention provides methods of generating compositions of EGCs, preferably CD34⁻CD133⁻CD73⁻CD14⁺CD11b⁺CXCR4⁺ cells. One such method comprises the steps of (i) culturing a cell population of cord blood, peripheral blood, or bone marrow on a substrate from about 2 to 48 hours, more preferably from 4-36 hours, more preferably from 8-24 hours, more preferably from 12-20 hours and more preferably for about 16 hours; and (ii) selecting adherent cells. In some embodiments, prior to culturing the cells on the substrate, the positive selection or a negative selection is performed. For example, a negative selection for cells expressing CD34⁻, CD133⁻, or CD73⁻, or combinations thereof, may be performed, such as by contacting the cells with an antibody that binds the marker and removing those cells which bind to the antibody.

Likewise, a positive selection may be performed for cells which expresses on or more of CD14, CD11b, or CXCR4, either in addition to or instead of the positive selection, such as by contacting the cells with an antibody that binds the marker and selecting those cells which bind to the antibody for culturing on the substrate.

One preferred method for isolating an EGC cell population comprises (i) contacting cells from a sample of umbilical cord blood or bone marrow or peripheral blood with a reagent that specifically binds CD133; (ii) removing from the sample cells that bind to the reagent to generate a sample enriched for CD133⁻ cells; (iii) culturing the cell sample on a substrate; and (iv) selecting adherent cells, thereby generating a population of EGC cells. In one embodiment, step (iii) comprises culturing the sample of enriched CD133⁻ cells in a tissue culture container. In another embodiment, step (iii) comprises culturing the sample of CD133⁻ cells for at least 1 hour. In another embodiment, step (iii) comprises culturing the sample of enriched CD133⁻ cells for at least 4 hours. In yet another embodiment, step (iii) comprises culturing the sample of enriched CD133⁻ cells for at least 12 hours. In another embodiment, step (iii) comprises culturing the sample of enriched CD133⁻ cells less than 24 hours. In another embodiment, step (iii) comprises culturing the sample of enriched CD133- cells less than 48 hours. In another embodiment, step (iii) comprises culturing the sample of enriched CD133- cells less than 7 days.

In one embodiment of the methods for isolating EGCs, the substrate is coated with an extracellular matrix protein. In one embodiment, the extracellular matrix protein is selected from the group consisting of fibronectin, collagen (including type I collagen or type IV collagen), laminin, fibronectin, laminin, hyaluronic acid, heparan sulfate, dermatan sulfate, sulfated proteoglycans, fibrin, elastin, and tenascin. In a preferred embodiment, the substrate is coated with fibronectin. In another embodiment, the substrate is coated with poly-D-lysine or alginate.

In another embodiment of the methods for isolating EGCs, the cells are cultured on the substrate using a cell culture medium having between 1% and 20% serum, preferably human serum. In another embodiment, the cell culture medium comprises less than 1% serum, while in other embodiments it is free, or substantially free, of serum. In another embodiment, the cell culture medium is free of exogenously added serum. In another embodiment, the culture medium comprises hEGF, hydrocortisone, human albumin, autologous plasma, VEGF, hFGF-B, heparin, IGF-1 and ascorbic acid.

In another embodiment of the methods for isolating EGCs that include the step of a negative and/or positive selection, the reagent that specifically binds to the cell-surface marker is an antibody, such as a monoclonal antibody. In other embodiments of the isolation methods, the population of adherent cells *i.e.* the EGCs, are cryopreserved.

The invention further provides compositions of EGCs generated according to any one of the methods described herein for their isolation.

Another aspect of the invention provides therapeutic methods for using EGCs either alone or in combination with other cells. One aspect of the invention provides methods of inducing neovascularization in a subject thereof, the method comprising administering to the subject a therapeutically effective amount of a composition comprising EGCs. In some embodiments, the EGCs are administered in combination with CD133⁺ cells. The CD133⁺ cells may be administered simultaneously to the subject, either as part of the same composition or as a separate composition. The CD133⁺ cells may also be administered before or after EGC administration. In preferred embodiments, the subject is afflicted with ischemia or vascular occlusion. In one embodiment, the ischemia is selected from the group consisting of cardiac, cerebral, peripheral vascular, and renal.

Another aspect of the invention includes methods of providing cellular therapies, wherein two cell populations are derived from umbilical cord blood and either combined into one composition or included in a kit as two compositions. One aspect provides a method of providing cellular therapies comprising: (a) purifying one or more cell populations from umbilical cord blood; (b) formulating a cryopreserved preparation including one or more of the cell populations; (c) providing a kit comprising the cryopreserved preparation to a medical care provider. In one embodiment, one of the cell populations is an enriched population of CD133⁺ cells, such as a population having at least 20%, 30%, 40%, 50%, 60%, 70%, 80% 90%, 95% 98%, 99% or about 100% CD133⁺ cells. In another embodiment, one of the cell populations comprises EGCs. In one embodiment, one of the cell populations is an enriched population of EGCs, such as a population having at least 20%, 30%, 40%, 50%, 60%, 70%, 80% 90%, 95% 98%, 99% or about 100% EGCs.

In yet another embodiment, one or more of the cell populations comprise mesenchymal stem cells (MSCs). In one embodiment, one of the cell populations is an enriched population of MSCs cells, such as a population having at least 20%, 30%, 40%, 50%, 60%, 70%, 80% 90%, 95% 98%, 99% or about 100% MSCs. In another embodiment, the kit comprises a buffer for thawing, rinsing or resuspending the cells. In another embodiment, the cellular therapy is ischemia or arterial thrombosis. In another embodiment, the ischemia is cardiac, renal, peripheral vascular, or cerebral ischemia.

In another embodiment, at least two cell populations are isolated from blood derived from a single umbilical cord. In another embodiment, the cryopreserved sample comprises at least one cell population isolated from a plurality of umbilical cord blood samples.

Another aspect of the invention provides methods of distributing a composition of EGCs, such as CD34⁻CD133⁻CD73⁻CD14⁺CD11b⁺CXCR4⁺ cells. One specific aspect of the invention provides a method for distributing a composition comprising EGCs, and optionally comprising CD133⁺ cells, for use by health care professionals, the method comprising placing the composition into a package under sterile conditions and distributing the package for use by health care professionals. The methods of distribution may be applied to the distribution of any one of the compositions described herein having EGCs, and may further be used in conjuction with the methods of generating compositions comprising EGCs.

### II. Definitions

For convenience, certain terms employed in the specification, examples, and appended claims, are collected here. Unless defined otherwise, all technical and scientific terms used herein have the same meaning as commonly understood by one of ordinary skill in the art to which this invention belongs.

The articles "a" and "an" are used herein to refer to one or to more than one (i.e., to at least one) of the grammatical object of the article. By way of example, "an element" means one element or more than one element.

The term "including" is used herein to mean, and is used interchangeably with, the phrase "including but not limited" to.

The term "or" is used herein to mean, and is used interchangeably with, the term "and/or," unless context clearly indicates otherwise.

The term "such as" is used herein to mean, and is used interchangeably, with the phrase "such as but not limited to".

The term "expression vector" and equivalent terms are used herein to mean a vector which is capable of inducing the expression of DNA that has been cloned into it after transformation into a host cell. The cloned DNA is usually placed under the control of (i.e., operably linked to) certain regulatory sequences such a promoters or enhancers. Promoters sequences maybe constitutive, inducible or repressible.

The term "operably linked" is used herein to mean molecular elements that are positioned in such a manner that enables them to carry out their normal functions. For example, a gene is operably linked to a promoter when its transcription is under the control of the promoter and, if the gene encodes a protein, such transcription produces the protein normally encoded by the gene. For example, a binding site for a transcriptional regulator is the to be operably linked to a promoter when transcription from the promoter is regulated by protein(s) binding to the binding site. Likewise, two protein domains are the to be operably linked in a protein when both domains are able to perform their normal functions. The term "encoding" comprises an RNA product resulting from transcription of a DNA molecule, a protein resulting from the translation of an RNA molecule, or a protein resulting from the transcription of a DNA molecule and the subsequent translation of the RNA product.

The term "promoter" is used herein to mean a DNA sequence that initiates the transcription of a gene. Promoters are typically found 5' to the gene and located proximal to the start codon. If a promoter is of the inducible type, then the rate of transcription increases in response to an inducer. Promoters may be operably linked to DNA binding elements that serve as binding sites for transcriptional regulators. The term "mammalian promoter" is used herein to mean promoters that are active in mammalian cells. Similarly, "prokaryotic promoter" refers to promoters active in prokaryotic cells.

The term "expression" is used herein to mean the process by which a polypeptide is produced from DNA. The process involves the transcription of the gene into mRNA and the translation of this mRNA into a polypeptide. Depending on the context in which used, "expression" may refer to the production of RNA, protein or both.

The term "recombinant" is used herein to mean any nucleic acid comprising sequences which are not adjacent in nature. A recombinant nucleic acid may be generated *in vitro*, for example by using the methods of molecular biology, or *in vivo*, for example by insertion of a nucleic acid at a novel chromosomal location by homologous or non-homologous recombination.

The terms "disorders" and "diseases" are used inclusively and refer to any deviation from the normal structure or function of any part, organ or system of the body (or any combination thereof). A specific disease is manifested by characteristic symptoms and signs, including biological, chemical and physical changes, and is often associated with a variety of other factors including, but not limited to, demographic, environmental, employment, genetic and medically historical factors. Certain characteristic signs, symptoms, and related factors can be quantitated through a variety of methods to yield important diagnostic information.

The term "prophylactic" or "therapeutic" treatment refers to administration to the subject of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "therapeutic effect" refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and conditions in an animal or human. The phrase "therapeutically-effective amount" means that amount of such a substance that produces some desired local or systemic effect at a reasonable benefit/risk ratio applicable to any treatment. In certain embodiments, a therapeutically-effective amount of a compound will depend on its therapeutic index, solubility, and the like. For example, certain compounds discovered by the methods of the present invention may be administered in a sufficient amount to produce a reasonable benefit/risk ratio applicable to such treatment.

The term "effective amount" refers to the amount of a therapeutic reagent that when administered to a subject by an appropriate dose and regime produces the desired result.

The term "subject in need of treatment for a disorder" is a subject diagnosed with that disorder or suspected of having that disorder.

The term "antibody" as used herein is intended to include whole antibodies, e.g., of any isotype (IgG, IgA, IgM, IgE, etc), and includes fragments thereof which are also specifically reactive with a vertebrate, e.g., mammalian, protein. Antibodies can be fragmented using conventional techniques and the fragments screened for utility and/or interaction with a specific epitope of interest. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The term antibody also includes polyclonal, monoclonal, or other purified preparations of antibodies and recombinant antibodies.

Other technical terms used herein have their ordinary meaning in the art that they are used, as exemplified by a variety of technical dictionaries, such as the McGraw-Hill Dictionary of Chemical Terms and the Stedman's Medical Dictionary.

### III. Compositions and Populations of CD34-CD133-CD73- Cells

The invention is based, in part, on a novel population of EGCs. This novel cell population may be used, for example, to treat ischemia and/or to promote vascularization in a subject in need thereof.

One aspect of the invention provides a composition comprising a population of human cells, wherein at least 10% of the cells are EGCs. In one embodiment, at least 20% of the cells in the composition are EGCs. In other embodiments, at least 25%, 30%, 35%, 40%, 45%, 50%, 55%, 60%, 65%, 70%, 75%, 80%, 85%, 90%, 95%, 96%, 97%, 98% or about 99% of the cells in the composition are EGCs, such as but not limited to, CD34⁻CD133⁻CD73⁻CD14⁺CD11b⁺CXCR4⁺ cells.

In preferred embodiments, EGCs are capable of growing in cell culture as adherent cells on fibronectin coated substrates, such as fibronectin-coated tissue culture dishes or flasks. In another embodiment, the EGCs grow as adherent cells on substrates coated with an extracellular matrix protein selected from the group consisting of fibronectin, collagen, laminin, fibronectin, laminin, hyaluronic acid, heparan sulfate, dermatan sulfate, sulfated proteoglycans, fibrin, elastin, and tenascin. In some embodiments, the collagen is type I collagen or type IV collagen. In yet another embodiment, EGCs of the present invention grow as adherent cells in substrates coated with poly-D-lysine or alginate.

In one embodiment of the methods described herein, the EGCs are capable of differentiating into stromal cells. In another embodiment, the EGCs are capable of differentiating into cells which express platelet endothelial cell adhesion molecule-1 (PECAM1), transcription factor GATA-2, N-cadherin, vascular endothelial-cadherin, von Willebrand factor, or combinations thereof.

EGCs may be isolated from umbilical cord blood. In another embodiment, EGCs are isolated from bone marrow, while in another embodiment they are isolated from peripheral blood. When a compositions comprising EGCs is used in the methods described herein for administration to a subject, the EGCs may be autologous, allogenic, or HLA-compatible with the subject. The cells may be isolated from the subject's own bone marrow, peripheral blood or even umbilical cord blood.

In some embodiments, the compositions comprise additional cell populations. In a preferred embodiment, the additional population is a population of CD133⁺ cells. One aspect of the invention provides a composition comprising two populations of cells, wherein the first population makes up at least 10% of the cells in the composition and consists of EGCs; and a second population which also makes at least 10% of the cells in the composition and consists of CD133⁺ cells. In one embodiment, the second population of CD133⁺ cells makes up at least 15%, 20%, 25%, 30%, 35%, 40%, 45%, 50%, 55%, 60%, 65%, 70%, 75%, 80%, 85%, 86%, 87%, 88%, 89% or 89.9% of the cells in the composition.

The cell surface marker CD133⁺ is also known as AC133 (Gehling et al., 2000, Blood. 95(10): 3106-12; Yin et al. 1997. Blood 90(12):5002-12; Buhring et al. 1999. Ann NY Acad SCI 99 872: 25-39; Majka et al. 2000. Folia Histochem Cytobiol. 38:53-63). Antibodies which recognize the CD133 antigen are described in U.S. Patent No. 5,843,633.

Methods and sources of isolating CD133⁺ cells are described, for example, in International PCT Application Nos. WO03/095631, WO99/37751, and WO01/94420, and U.S. Patent Publication Nos. 2003/0091547, 2003/0199464 and 2002/0051762

The CD133⁺ cells in the composition may be CD133⁺CD34⁺ cells. The CD133⁺ cells in the composition may be CD133⁺CD34⁻ cells. CD133⁺ hematopoietic stem cells are of particular interest in studies directed to therapeutic angiogenesis, as these cells have been shown to differentiate into endothelial cells after short-term culturing. In the compositions described herein, the CD133⁺ cells may be CD31⁺, CD34⁺, CD146⁺, CD133⁺, VE-cadherin⁺, or a combination thereof. The CD133⁺ cells may also express one or more of the following markers, preferably at the given frequencies: CD34 (75%-99%), KDR (EGFR2) (0-10%), CD105 (15%-30%) and CXCR4 (2%-15%).

The CD133⁺ cells may be cells which have not undergone appreciable culturing in vitro, such as cells that have been cultured for less than 12, 8 or more preferably less than 4 hours. In another embodiment, the CD133⁺ cells in the composition have been propagated *in vitro*. In a specific embodiment, the population of CD133⁺ cells is expanded *in vitro* under conditions that promote the formation of endothelial cells. Several culture media suitable for promoting endothelial cell differentiation are known. As a non-limiting example, a suitable medium is described in Kalka et al. (2000) PNAS 97: 3422-3427, is EC basal medium-2 (EBM-2) (Clonetics, San Diego). This medium has 5% serum, such as fetal bovine serum (FBS), and standard SingleQuot^{™} additives that include human VEGF-1, human basic fibroblast growth factor-2 (FGF), insulin-like growth factor-1 (IGF-1), hydrocortisone, ascorbic acid and heparin. In another embodiment, CD133⁺ cells are cultured in hematopoetic cell culture media comprising antioxidants, albumin, lipid agent, insulin, transferrin, trace elements and glucocorticoid(s), such as is described in U.S. Patent No. 6,733,746. CD133⁺ cells may be isolated as described in U.S. Pub. No. 2005/0069527.

In another embodiments of the EGC compositions having additional cell populations, the additional cell population is a mesenchymal stem cells (MSCs) population. One aspect of the invention provides a composition comprising two populations of cells, wherein the first population makes up at least 10% of the cells in the composition and consists of EGCs; and a second population which also makes at least 10% of the cells in the composition and consists of MSCs. In one embodiment, the second population ofMSCs makes up at least 15%, 20%, 25%, 30%, 35%, 40%, 45%, 50%, 55%, 60%, 65%, 70%, 75%, 80%, 85%, 86%, 87%, 88%, 89% or 59.9% of the cells in the composition.

Mesenchymal stem cells are the formative pluripotent blast cells found in the bone marrow and peripheral blood that are capable of differentiating into any of the specific types of connective tissues (*i.e.*, the tissues of the adipose, areolar, osseous, cartilaginous, elastic, and fibrous connective tissues) depending upon various environmental influences. Applicants have previously reported extensive research on methods to isolate, culture-expand and phenotypically characterize hMSCs, as well as their multi-lineage developmental potential and capacity to regulate a variety of other developmental events including angiogenesis (Fleming, JE Jr. et al. Dev. Dyn. 212, 119-132 (1998); Barry FP et al. Biochem. Biophys. Res. Commun. 265, 134-139 (1999)). Although hMSCs are rare, comprising about 0.01-0.0001 % of the total nucleated cells of bone marrow, cell culture methodology have been developed for their isolation from bone marrow, purification to homogeneity from other bone marrow cells and mitotic expansion in culture without loss of their stem cell potential (Haynesworth SE et al. Bone 13, 81-88 (1992)). Human adult MSC, although marrow-derived, do not express CD34 or CD45, but have been shown to express IL-6, -7, -8, -11, -12, -14, -15, M-CSF, flt-3 ligand (FL), and SCF in steady state, and do not express IL-3 and TGFβ. Exposure to dexamethasone results in decreased expression of LIF, IL-6 and IL-11 (Haynesworth SE et al. J. Cell Physiol. 166, 585-592 (1996)). Moreover, adhesion molecules expressed by stromal cells of importance in supporting early hemangioblasts, include fibulin-1 and fibulin-2, tenascin-C, stromal cell-derived factor 1 (SDF-1), and collagen type VI.

Mesenchymal stem cells can be isolated from peripheral blood or bone marrow. A method for preparing hMSC has been described in U.S. Patent No. 5,486,359. Furthermore, mesenchymal stem cells may also be isolated from umbilical cord blood, as described by Erices et al. 2000 Br. J Haematol 109(1):235-42. MSCs may be isolated from peripheral blood, such as is described in U.S. Patent No. 6,261,549.

Several techniques are known for the rapid isolation of mesenchymal stem cells including, but are not limited to, leucopheresis, density gradient fractionation, immunoselection, differential adhesion separation, and the like. For example, immunoselection can include isolation of a population of hMSCs using monoclonal antibodies raised against surface antigens expressed by bone marrow-derived hMSCs, *i.e.*, SH2, SH3 or SH4, as described, for example, in U.S. Patent No. 6,387,367. The SH2 antibody binds to endoglin (CD105), while SH3 and SH4 bind CD73. Accordingly, in a preferred embodiment of the compositions and related methods described herein, the MSCs are CD73⁺CD105⁺ cells. In another embodiment, the MSCs are CD45⁺CD73⁺CD105⁺ cells.

Further, these monoclonal antibodies provide effective probes which can be utilized for identifying, quantifying and purifying hMSC, regardless of their source in the body. In one embodiment of the methods described herein, mesenchymal stem cells are culture expanded to enrich for cells expressing CD45, CD73, CD105, stro-1, or a combination thereof. In another embodiment, human mesenchymal stem cells are culture-expanded to enrich for cells containing surface antigens identified by monoclonal antibodies SH2, SH3 or SH4. A stro-1 antibody is described in Gronthos et al., 1996, J. Hematother. 5: 15-23. Further cell surface markers that may be used to enrich for human mesenchymal stem cells include those listed in Table I, page 237 of Fibbe et al., 2003. Ann. N.Y. Acad. Sci. 996: 235-244.

Human MSC can be maintained in culture media which can be chemically defined serum free media or can be a "complete medium", such as Dulbecco's Modified Eagles Medium supplemented with 10% serum (DMEM). Suitable chemically defined serum free media are described in U.S. Patent No. 5,908,782 and in international PCT Publication No. WO96/39487, and complete media is described in U.S. Patent No. 5,486,359. Chemically defined medium comprises a minimum essential medium such as Iscove's Modified Dulbecco's Medium (IMDM), supplemented with human serum albumin, human Ex Cyte lipoprotein, transferrin, insulin, vitamins, essential and non-essential amino acides, sodium pyruvate, glutamine and a mitogen. These media stimulate mesenchymal stem cell growth without differentiation. Culture for about 2 weeks results in 10 to 14 doublings of the population of adherent cells. After plating the cells, removal of non-adherent cells by changes of medium every 3 to 4 days results in a highly purified culture of adherent cells that have retained their stem cell characteristics, and can be identified and quantified by their expression of cell surface antigens identified by monoclonal antibodies SH2, SH3 and/or SH4.

The two cell populations in the EGC compositions may be derived from the same source or from different sources. For instance, EGCs may be isolated from umbilical cord blood, while the MSCs or CD133⁺ cells may be derived from peripheral blood or from bone marrow. In other embodiments, the two cell populations are derived from the same individual, while in other embodiments they may be isolated from different individual. For example, if a first cell population is isolated from the bone marrow of a first individual, the second cell population may be isolated from a bone marrow sample of the first or of a second individual, from a peripheral blood sample of the first or of a second individual, or from the umbilical cord blood of the first or of a second individual. Accordingly, in some embodiments, the two cell populations are autologous with each other while in other embodiments they are allogenic. In one preferred embodiment, both the EGCs and the CD133⁺ cells are isolated from umbilical cord blood, while in another preferred embodiment the EGCs are isolated from peripheral blood or from bone marrow and the CD133⁺ cells are isolated from cord blood.

In some embodiments of the compositions described herein, at least one cell in a population, such as the EGC cell population, a CD133⁺ cell population, or an MSC population, is genetically modified. In other embodiments, at least 0.5%, 1%, 5%, 10%, 20%, 50%, 80%, 90%, 95% or 100% of the cells in the population are genetically modified. In some embodiments, the cells are genetically-modified to express a recombinant polypeptide, while in other embodiments the cells are genetically modified to express a double stranded RNA molecule, such as a hairpin RNA molecule, which inhibits the function of an endogenous gene. Preferred recombinant polypeptides include growth factors, chemokines, antibodies, cytokines, or receptors which bind to growth factors, chemokines, or cytokines.

In another embodiment, the recombinant peptide is vascular endothelial growth factor (VEGF), hepatocyte growth factor (HGF), fibroblast growth factor (FGF), stromal cell-derived factor 1 (SDF-1), angiopoietin-1 or interleukin 8 (IL-8). In another embodiment, the recombinant polypeptide an angiogenic polypeptide, such as acidic fibroblast growth factor (aFGF), basic fibroblast growth factor (bFGF), vascular endothelial growth factor (VEGF-1), epidermal growth factor (EGF), transforming growth factor α and β (TGF-α and TFG-β), platelet-derived endothelial growth factor (PD-ECGF), platelet-derived growth factor (PDGF), tumor necrosis factor α (TNF-α), hepatocyte growth factor (HGF), insulin like growth factor (IGF), erythropoietin, colony stimulating factor (CSF), macrophage CSF (M-CSF), angiopoetin-1 (Ang1) or nitric oxide synthetase (NOS); or an effective fragment thereof. In yet another embodiment, the recombinant polypeptide is CXCR-4, CXCR-5, VEGF-B, VEGF-C, VEGF-2, VEGF-3; or an effective fragment thereof.

In another embodiment, the genetic modification promotes angiogenesis, vasculogenesis, or both. In another embodiment, the genetic modification reduces inflammation or coagulation. In another embodiment, the recombinant polypeptide is selected from the group consisting of leukemia inhibitory factor, IL-1 through IL-13, IL-15 through IL-17, IL-19 through IL-22, granulocyte macrophage colony stimulating factor (GM-CSF), granulocyte colony stimulating factor (G-CSF), macrophage colony stimulating factor (M-CSF), erythropoietin (Epo), thrombopoietin (Tpo), Flt3-ligand, B cell activating factor, artemin, bone morphogenic protein factors, epidermal growth factor (EGF), glial derived neurotrophic factor, lymphotactin, macrophage inflammatory proteins, myostatin, neurturin, nerve growth factors, platelet derived growth factors, placental growth factor, pleiotrophin, stem cell factor, stem cell growth factors, transforming growth factors, tumor necrosis factors, Vascular Endothelial Cell Growth Factors, and fibroblast growth factors, FGF-acidic and basic fibroblast growth factor. In another embodiment, the recombinant polypeptide expressed by the genetically-modified cells promotes the proliferation, the differentiation or the ability of the cells in which it is produced, or in other cells present in the compositions, to localize to the ischemic tissue. In another embodiments, the genetic modification enhances the ability of the modified cells to interact with cells at the site of the ischemic tissue.

Cells may be genetically modified, for example, using the methods commonly known in the art, such as by transfection, transformation or transduction, using recombinant expression vectors (see Examples and Sambrook et al., Molecular Cloning: A Laboratory Manual, 2nd ed., Cold Spring Harbor Press, Plainview, N.Y. (1989); Ausubel et al., Current Protocols in Molecular Biology (Supplement 47), John Wiley & Sons, New York (1999)). The vector may be integrated into chromosomal DNA or be carried as a resident plasmid by the genetically modified cells. In some embodiments, retroviruses are used to genetically modify the cells. Additional genes that may be introduced into the cells are described in International PCT Publication No. WO99/37751.

In a related embodiment, one or more of the cell populations may be non-genetically modified, such as by exposure to antibodies which may adhere to the cell surface. In some embodiments, this treatment is intended to increase the localization of the modified cells to the ischemic tissue. In a related embodiment, at least one polypeptide or an organic molecule is coupled to the surface of at least one of the cell populations, such as the EGCs. The terms "coupled" or "coupling" refer to a chemical, enzymatic or other means (e.g. antibody) by which a polypeptide is linked to a cell such that the polypeptide is present on the surface of the cell. For example, the polypeptide can be chemically crosslinked to the cell surface using commercially available crosslinking reagents (Pierce, Rockford Ill.). Another approach to coupling a polypeptide to a cell is to use a bispecific antibody which binds both the polypeptide and a cell-surface molecule on the cell.

In some embodiments, the compositions further comprises a physiologically acceptable medium or a pharmaceutically acceptable medium. In some preferred embodiments, the compositions provided herein are formulated as injectable compositions. In some embodiments, the compositions provided herein further comprise an additional component, such as a biologically active component. In a specific embodiment, the component is human serum, preferably human serum from the subject for which administrations of the cells is intended. In another embodiment, the additional component may comprise a component of human serum, such as human serum albumin. Human serum albumin may be purified from natural sources or may be produced by recombinant techniques. In another embodiment, the additional component comprises a preservative, such as citrate phosphate dextrose adenine (CPDA) or heparin. In another embodiment, the additional component is soluble human fibronectin or another extracellular matrix protein or soluble fragment thereof. In another embodiment, the compositions comprise at least one-cytokine, chemokine or growth factor. In one embodiment, the growth factor is selected from the group consisting of flt3 ligand, kit ligand, thrombopoietin, basic fibroblast growth factor, interleukin 6, interleukin 11, interleukin 3, granulomonocytic colony-stimulatory factor, granulocytic colony-stimulatory factor, monocytic colony-stimulatory factor, erythropoietin, angiopoietin, and hepatocyte growth factor. Exemplary growth factors include bFGF and VEGF. In preferred embodiments, the cytokine, chemokine or growth factor promotes angiogenesis or cardiovascularization. In other embodiments, the compositions comprise synthetic compounds which promotes angiogenesis or cardiovascularization or which inhibit inflammation or coagulation.

In some embodiments of the compositions provided herein, the compositions are frozen or cryopreserved. In other embodiments, the composition is a desiccated population of cells. In some embodiments, the compositions comprises at least one carbohydrate, such as trehalose. The tetrahalose may be present at a concentration of at least 25nM. Methods for generating desiccated cell populations is described, for example, in U.S. Patent No. 6,528,309.

The invention further provides kits comprising any of the compositions described herein. In one specific embodiment, the kit comprises a cryopreserved composition. In some preferred embodiments, the kit comprises EGCs in combination with MSCs or CD133⁺ cells. In some embodiments, the kit comprises an excipient, such as an excipient suitable for injection. Preferred excipients include nonpyrogenic excipients. In another embodiment, the kit comprises at least one component of a closed sterile system suitable for intra-coronary injection. Exemplary components include a needle, syringe, catheter-based syringe, needle based injection device or needle-less injection device. In another embodiment, the kit further comprises at least one anticoagulating agent. Exemplary anticoagulating agents include inhibitors of GP IIb-IIIa, thrombin or a vitamin K-dependent coagulation factor. In a specific embodiment, the anticoagulating agent is selected from the group consisting of eptifibatide, argatroban, ximelagatran, warfarin and heparin.

Another aspect of the invention provides a kit for inducing formation of new blood vessels in ischemic tissue of a mammal, wherein the kit comprises EGCs and optionally comprises (i) at least one angiogenic protein; or (ii) a pharmacologically acceptable carrier solution, or (iii) both.

### IV. Methods of Isolating EGCs

The invention further provides methods of isolating EGCs. One aspect of the invention provides methods of generating compositions of EGCs, preferably CD34⁻ CD133⁻CD73⁻CD14⁺CD11b⁺CXCR4⁺ cells. One such method comprises culturing a cell population of umbilical cord blood, peripheral blood, or bone marrow, on a substrate for about 2-48 hours, more preferably for 4-36 hours, more preferably for 8-24 hours, more preferably for 12-20 hours and more preferably for about 16 hours; and selecting cells that adhere to the substrate. In one embodiment, the cells is cultured on a substrate for at least one hour. In another embodiment, the cells are cultured for at least 4 hours, 12 hours, 24 hours, 48 hours, 4 days or 7 days. In an exemplary embodiment, cells are cultured from about 12 to about 36 hours. In another exemplary embodiment, the cells are cultured for less than about 24 hours. In some embodiments, the substrate does not contain immobilized antibodies.

In some embodiments, prior to culturing the cells on the substrate, the positive selection or a negative selection is performed. For example, a negative selection for cells expressing CD34⁻, CD133⁻, or CD73⁻, or combinations thereof, may be performed, by contacting the cells with a agent that binds the marker, and removing those cells which bind to the agent. For example, monoclonal antibodies which recognize the CD133 antigen are described in U.S. Patent No. 5,843,633. Likewise, a positive selection may be performed for cells which expresses on or more of CD14, CD11b, or CXCR4, either in addition to or instead of the positive selection, such as by contacting the cells with an antibody that binds the marker and selecting those cells which bind to the agent for culturing.

One preferred method for isolating an EGC cell population comprises (i) contacting cells from a sample of umbilical cord blood or bone marrow or peripheral blood with a reagent that specifically binds CD133; (ii) removing from the sample cells that bind to the reagent to generate a sample enriched for CD133⁻ cells; (iii) culturing the cell sample on a substrate; and (iv) selecting adherent cells, thereby generating a population of EGC cells. In one embodiment, the sample of enriched CD133⁻ cells is cultured in a tissue culture container. In another embodiment, step (iii) comprises culturing the sample of CD133⁻ cells for at least 1 hour. In another embodiment, step (iii) comprises culturing the sample of enriched CD133⁻ cells for at least 4 hours. In yet another embodiment, step (iii) comprises culturing the sample of enriched CD133⁻ cells for at least 12 hours. In another embodiment, step (iii) comprises culturing the sample of enriched CD133⁻ cells less than 24 hours. In another embodiment, step (iii) comprises culturing the sample of enriched CD 133-cells less than 48 hours. In another embodiment, step (iii) comprises culturing the sample of enriched CD133- cells less than 7 days. In one preferred embodiments, the enriched sample is cultured for about 16 hours.

The exposure of the cell mixture to monoclonal antibodies for positive or negative selection can be performed in solution, as is the case with fluorescence-activated cell sorting, or it can be with the monoclonal antibody immobilized on a solid support, such as is the case with column chromatography or direct immune adherence. In addition, a combination of soluble and solid support monoclonal antibodies can be used to expose the cell mixture to such monoclonal antibodies. The mixture of cells that is to be exposed to the monoclonal antibody can be any sample of bone marrow cells, blood cells or umbilical cord blood. After exposure of the cell sample to the monoclonal antibody, those cells with CD133 on their cell surfaces will bind to the monoclonal antibody to form an antibody-CD133 receptorcell complex. Such CD133 receptor cell complexes can then be separated from noncomplexed cells by methods that are known in the art. Preferred methods of separation include column chromatography, fluorescence-activated cell sorting, magnetic bead separation, and direct immune adherence. CD133⁺ cells can thus be removed from the sample resulting in an enriched sample of CD133- cells.

In some embodiments, EGCs may be further purified based on the expression, or lack of expression, of one, two or more additional cell surface molecule, such as CD34. For example, a population comprising EGCs may be further purified by selecting out cells that express CD34. Purification may be performed by means of an anti-CD34 antibody, such as the anti-My-10 monoclonal antibody described in U.S. Patent No. 5,130,144. The hybridoma cell line that expresses the anti-My monoclonal antibody is available from the American Type Culture Collection, 12301 Parklawn Drive, Rockville, Maryland 20852, USA. Some additional sources of antibodies capable of selecting CD34⁺ cells include AMAC, Westbrook, Maine; Coulter, Hialea, Florida; and Becton Dickinson, Mountain View, California. CD34⁺ cells may also be isolated by means of comparable antibodies, which may be produced by methods known in the art, such as those described in U.S. Patent No. 5,130,144. Other markers that may be used in a negative or a positive selection, include, but are not limited to CD1, CD2, CD3, CD4, CD5, CD8, CD10, CD11b, CD 13, CD14, CD15, CD16, CD19, CD20, CD24, CD25, CD28, CD29, CD33, CD36, CD38, CD41, CD41, CD56, CD66b, CD66e, CD69 or glycophorin A. In some preferred embodiments, the cells are further positively selected for the expression of CD14, CD11b or CXCR4.

Cells may be cultured in any container known in the art, such as plastic petri plates, multi-well plates, tissue culture containers and flasks. In a preferred embodiment, the substrate, or surface, on which the cells are grown is coated with an extracellular matrix protein. In one embodiment, the extracellular matrix protein is selected from the group consisting of fibronectin, collagen, laminin, fibronectin, laminin, hyaluronic acid, heparan sulfate, dermatan sulfate, sulfated proteoglycans, fibrin, elastin, and tenascin. In a preferred embodiment, the collagen is type I collagen or type IV collagen. In another embodiment, the substrate is coated with poly-D-lysine or alginate. In a preferred embodiment, the substrate is coated with a human fibronectin .

The cells may be cultured in media lacking serum. Serum-free culture media has been described for the culturing of bone marrow derived cells in U.S. Patent Nos. 6,037,174, 5,766,951, 6,224,860 and 6,372,210. In other embodiments, the sample of cells is cultured in cell culture medium comprising between 1 and 20% serum. In preferred embodiments, the serum is human serum, such as from a subject who is to be the recipient of a transfusion of the cultured cells. In another embodiment, the cell sample is cultured in cell culture medium comprising hEGF, hydrocortisone, human albumin, autologous plasma, VEGF, hFGF-B, heparin, IGF-1 and ascorbic acid.

In some embodiments, the methods further comprise the step of cryopreserving the adherent cells. Methods of cryopreservation of cells are well-known in the art (see for example U.S. Patent No. 5,759,764).

The invention provides also provides a method of isolating a therapeutic composition of cells, the method comprising (i) contacting cells from a sample of umbilical cord blood or bone marrow or peripheral blood with a reagent that specifically binds CD133; (ii) removing from the sample cells that bind to the reagent to generate a sample enriched for CD133⁻ cells; (iii) culturing the cell sample on a substrate; (iv) selecting the adherent cells; and (v) combining the adherent cells of step (iv) with the cells that were removed in step (ii), thereby generating a therapeutic composition of cells.

The invention further provides a population comprising EGCs which are isolated according to any of the methods described herein.

### V. Methods of Treating Ischemia

Some aspects of the invention provide methods of treating or preventing a disorder and methods of using EGCs in the manufacture of medicaments for the treatment of ischemia. Some aspects provide methods of preventing disorders which are associated with ischemia, vascular occlusion, reduced blood circulation or reduced vascularization. One aspect of the invention provides methods for inducing neovascularization in a subject in need thereof. Numerous conditions may cause a subject to be in need of neovascularization. For example, the subject may have a wound that requires healing. The wound may be an acute wound, such as those caused by bums and contact with hard and/or sharp objects. For example, patients recovering from surgery, such as cardiovascular surgery, cardiovascular angioplasty, carotid angioplasty, and coronary angioplasty all require neovascularization. The wound may also be a chronic wound. Some examples of chronic wounds include ulcers, such as vascular ulcers and diabetic ulcers. The cells of the present invention may be used in increasing cardiac or peripheral (i.e. limb) vascularization. Therefore, the methods of the present invention are especially desirable in treating cardiac and peripheral ischemia. Patients suffering from other conditions also require neovascularization. Such conditions include sickle cell anemia and thalassemia.

One aspect of the invention provides a method of inducing neovascularization in a subject in need thereof, the method comprising administering to the subject a therapeutically effective amount of any one of the compositions comprising EGCs described herein. A related aspect provides for the use of any of the EGC compositions described herein for the manufacture of a medicament for inducing neovascularization in a subject in need thereof.

In one embodiment of the methods described herein for the inducement of neovascularization in a subject, the subject is afflicted with ischemia. The present methods are not limited to ischemia in any particular tissue, but are applicable to any type of ischemia. For example, in one embodiment, the subject suffers from ischemia in multiple tissues. In such embodiment, a systemic infusion of cells to the subject may be performed, or alternatively or in combination, one or more localized infusions near the ischemic tissue may be performed. In one embodiment of the methods described herein, the ischemic myocardium comprises an area of viable myocardium. In a related embodiment, the ischemia is selected from the group consisting of cerebrovascular ischemia, renal ischemia, pulmonary ischemia, limb ischemia, ischemic cardiomyopathy and myocardial ischemia.

In one embodiment of the methods described herein, the subject afflicted with an ischemic tissue is in need of treatment for chronic myocardial ischemia. In one specific embodiment of the methods described herein for the treatment of ischemia, the ischemia is selected from the group consisting of cardiac, peripheral vascular, cerebral and renal ischemia. In a specific embodiment, the subject is afflicted with at least one ischemic condition selected from the group consisting of myocardial infarction, angina pectoris, any cardiac surgical interventions, circulatory insufficiency in extremities, ischemia-reperfusion injury, stroke, trauma and peripheral vascular disease (PVD).

In one embodiments of the ongoing methods, the compositions that are administered to the subject comprise a population of human cells, wherein at least 10% of the cells are EGCs. In another embodiment, the compositions comprise a population of human cells, wherein at least 10% of the cells are EGCs and wherein at least 10% of the cells are CD133⁺. In another embodiment, the compositions comprise a population of human cells, wherein at least 10% of the cells are EGCs and wherein at least 10% of the cells are MSCs. In yet another embodiment, the compositions comprise a population of human cells, wherein at least 10% of the cells are EGCs, wherein at least at least 10% of the cells are CD133⁺, and wherein at least 10% of the cells are MSCs.

In specific embodiments of the methods described herein for inducing neovascularization, the cells that are administered to the subject are autologous, allogenic, or HLA compatible with the subject. In some embodiments, some of the cells that are administered to the subject are allogenic and some are autologous. The number of EGCs administered to an individual afflicted with an ischemic tissue will vary according to the severity of the ischemia, the size of the tissue that is ischemic, and the method of delivery. In one embodiment of the methods described herein, the therapeutically effective amount of EGCs is a safe and effective amount. In another specific embodiment, the amount of each cell type is at least 1 x 10⁴ cells. In another embodiment, the amount of each cell type that is administered to the subject is between about 10⁴ and about 5 x 10⁸ cells. The amount of cells administered to the subject will depend on the mode of administration and the site of administration. For example, a therapeutically effective cell dose via intracoronary injection (or intra-renal or intra-carotid) may be lower than that for intra-femoral injection. When two types of cells are administered to the subject, such as when EGCs and CD133⁺ cells are administered, the ratio of the two cell types may be, for example, from about 20:1 to about 1:20, from about 10:1 to about 1:10, from about 5:1 to about 1:5, or from about 2:1 to about 1:2.

In some embodiments of the methods described herein, at least one recombinant polypeptide or at least one drug, agent or polypeptide, such as a recombinant polypeptide, is further administered to the subject, either as part of the composition or administered separately. In one embodiment, the recombinant polypeptide comprises a growth factor, a chemokine, a cytokine, or receptors. In preferred embodiments, the recombinant polypeptide promotes angiogenesis, vasculogenesis, or both. Exemplary angiogenic factors include, but are not limited to, basic fibroblast growth factor, acidic fibroblast growth factor, vascular endothelial growth factor, angiogenin, transforming growth factor α and β, tumor necrosis factor, angiopoietin, platelet-derived growth factor, placental growth factor, hepatocyte growth factor, and proliferin. In another embodiment, the drug, agent or polypeptide is a thrombolytic agents, which include, but are not limited to, urokinase plasminogen activator, urokinase, streptokinase, inhibitors of α2-plasmin inhibitor, and inhibitors of plasminogen activator inhibitor-1, angiotensin converting enzyme (ACE) inhibitors, spironolactone, tissue plasminogen activator (tPA), an inhibitor of interleukin 1β converting enzyme, anti-thrombin III, and the like.

In some embodiments, the drug, agent or polypeptide promotes the proliferation, the differentiation or the ability of the EGCs, CD133⁺ cells, or MSCs, to localize to the ischemic tissue or to interact with cells from the ischemic tissue. In specific embodiments, the recombinant polypeptide comprises VEGF, BFGF, SDF, CXCR-4 or CXCR-5. In other embodiments, the drug, agent or polypeptides comprises an anticoagulant, such as an inhibitor of GP IIb-IIIa, thrombin or a vitamin K-dependent coagulation factor. In a specific embodiment the anticoagulating agent is selected from the group consisting of eptifibatide, argatroban, ximelagatran, warfarin, heparin, hirudin, tick anti-coagulant peptide, low molecular weight heparins such as enoxaparin, dalteparin, and ardeparin, ticlopidine, danaparoid, abciximab and tirofiban.

In other embodiments, the drug, agent or polypeptides has antiinflammatory properties. Antiinflammatory agents include, but are not limited to, any known nonsteroidal antiinflammatory agent, and any known steroidal antiinflammatory agent. Antiinflammatory agents include, but are not limited to, any known nonsteroidal antiinflammatory agent such as, salicylic acid derivatives (aspirin), paraaminophenol derivatives(acetaminophen), indole and indene acetic acids (indomethacin), heteroaryl acetic acids (ketorolac), arylpropionic acids (ibuprofen), anthranilic acids (mefenamic acid), enolic acids (oxicams) and alkanones (nabumetone) and any known steroidal antiinflammatory agent which include corticosteriods and biologically active synthetic analogs with respect to their relative glucocorticoid (metabolic) and mineralocorticoid (electrolyte-regulating) activities. Additionally, other drugs used in the therapy of inflammation or antiinflammatory agents including the autocoid antagonists such as all histamine and bradykinin receptor antagonists, leukotriene and prostaglandin receptor antagonists, and platelet activating factor receptor antagonists. In another embodiment, the polypeptide coadministered with the compositions is GM-CSF.

In one embodiment of the methods described herein, the drug, agent or polypeptide is administered to the subject in combination with the composition comprising the cells. The polypeptide or drug may be administered to the subject before, concurrently, or after the administration of the cells.

In the methods described herein, the therapeutically effective amount of EGCs, and the therapeutically effective amount of additional cell populations, can range from the maximum number of cells that is safely received by the subject to the minimum number of cells necessary for either induction of new blood vessel formation in the ischemic tissue or for increasing blood flow to the ischemic tissue. Generally, the therapeutically effective amount of each cell population is at least 1 x 10⁴ per kg of body weight of the subject and, most generally, need not be more than 7 x 10⁵ of each type of cell per kg. The ratio of EGCs to CD133⁺ cells or to MSCs can vary from about 5:1 to about 1:5. A ratio of about 1:1 is preferable. Although it is preferable that the cell populations be autologous or HLA-compatible with the subject, the cell populations can be isolated from other individuals or species or from genetically-engineered inbred donor strains, or from *in vitro* cell cultures.

The therapeutically effective amount of the EGCs and/or other cell populations can be suspended in a pharmaceutically acceptable carrier or excipient. Such a carrier includes but is not limited to basal culture medium plus 1% serum albumin, saline, buffered saline, dextrose, water, and combinations thereof. The formulation should suit the mode of administration. Accordingly, the invention provides use of EGCs, for the manufacture of a medicament to treat an ischemic tissue or vascular occlusion in a subject in need thereof. In some embodiments, the medicament further comprises recombinant polypeptides, such as growth factors, chemokines or cytokines. In further embodiments, the medicaments comprise hMSCs or CD133⁺ cells. The cells used to manufacture the medicaments may be isolated, derived, or enriched using any of the variations provided for the methods described herein.

In a preferred embodiment, the composition of EGCs is formulated in accordance with routine procedures as a pharmaceutical composition adapted for intravenous administration to human beings. Typically, compositions for intravenous, intra-arterial or intracardiac administration are solutions in sterile isotonic aqueous buffer. Where necessary, the composition may also include a local anesthetic to ameliorate any pain at the site of the injection. Generally, the ingredients are supplied either separately or mixed together in unit dosage form, for example, as a cryopreserved concentrate in a hermetically sealed container such as an ampoule indicating the quantity of active agent. When the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

A variety of means for administering cells to subjects will, in view of this specification, be apparent to those of skill in the art. Such methods include injection of the cells into a target site in a subject. Cells may be inserted into a delivery device which facilitates introduction by injection or implantation into the subjects. Such delivery devices may include tubes, e.g., catheters, for injecting cells and fluids into the body of a recipient subject. In a preferred embodiment, the tubes additionally have a needle, e.g., a syringe, through which the cells of the invention can be introduced into the subject at a desired location. In a preferred embodiment, cells are formulated for administration into a blood vessel via a catheter (where the term "catheter" is intended to include any of the various tube-like systems for delivery of substances to a blood vessel). The cells may be prepared for delivery in a variety of different forms. For example, the cells may be suspended in a solution or gel. Cells may be mixed with a pharmaceutically acceptable carrier or diluent in which the cells of the invention remain viable. Pharmaceutically acceptable carriers and diluents include saline, aqueous buffer solutions, solvents and/or dispersion media. The use of such carriers and diluents is well known in the art. The solution is preferably sterile and fluid, and will often be isotonic. Preferably, the solution is stable under the conditions of manufacture and storage and preserved against the contaminating action of microorganisms such as bacteria and fungi through the use of, for example, parabens, chlorobutanol, phenol, ascorbic acid, thimerosal, and the like.

Modes of administration of the EGCs include but are not limited to systemic intracardiac, intracoronary, intravenous or intra-arterial injection and injection directly into the tissue at the intended site of activity. The preparation can be administered by any convenient route, for example by infusion or bolus injection and can be administered together with other biologically active agents. Administration is preferably systemic. Most preferably, the site of administration is close to or nearest the intended site of activity. In cases when a subject suffers from global ischemia, a systemic administration, such as intravenous administration, is preferred. Without intending to be bound by mechanism, generating cells such as EGCs and the CD133⁺ cells will, when administered, migrate or home to the ischemic tissue in response to chemotactic factors produced due to the injury.

In one embodiment, the EGCs are co-administered simultaneously with the other cell populations. In another embodiment the other cell populations are administered before or after the injection of the EGCs. Administration of EGCs and other cell populations may be carried out using the same mode or different modes of administration. For example, EGCs can be administered by intracoronary injection, while CD133⁺ cells might be administered intravenously.

In some embodiments of the methods described herein, the cells which are to be administered to the subject are incubated in a buffer, such as a saline buffer. In one preferred embodiment, the buffer comprises human blood serum isolated from the same subject who is the recipient of the therapy. Human serum may be isolated using standard procedures. A solution comprising human blood serum may also be used to thaw a sample of cells that has been cryopreserved. In some embodiments, the solution comprising human serum comprises between 1-20% human serum, or more preferably between 5-15%.

In embodiments of the methods described herein, administering to the subject comprises an infusion of cells into the subject. The infusion may comprise a systemic infusion of cells into the subject, or it may comprise an infusion of cells in the proximity of the ischemic tissue, so as to facilitate the migration of cells to the ischemic tissue. The infusion may also be performed on the blood vessels that supply blood to the ischemic tissue, or to blood vessels which remove blood from the ischemic tissue. In specific embodiments of the methods described herein, the infusion of cells into the subject comprises an infusion into bone marrow, an intra-arterial infusion, an intramuscular infusion, an intracardiac infusion, and intracoronary infusion, an intravenous infusion or an intradermal infusion. In one embodiment of the methods described herein, the EGCs cells are administered to the subject by infusion into at least one coronary artery or coronary vein. In a specific embodiments of the methods described herein, the coronary artery is an epicardial vessel that provides collateral blood flow to the ischemic myocardium in the distribution of a chronic totally occluded vessel.

In some embodiments of the methods described herein, administration of the cells to the subject is performed using an intra-arterial catheter, such as but not limited to a balloon catheter, or by using a stent. Any method currently available for delivering cells to a subject may be used.

In particular, the invention methods described herein for inducing neovascularization are useful for therapeutic vasculogenesis for the treatment of myocardial ischemia in humans. Administration of the compositions described hereon for the induction of neovascularization can be used as a sole treatment or as an adjunct to surgical and/or medical treatment modalities. For example, the methods described herein for treatment of myocardial ischemia can be used in conjunction with coronary artery bypass grafting or percutaneous coronary interventions. The methods described herein are particularly useful for subjects that have incomplete revascularization of the ischemic area after surgical treatments and, therefore, have areas of ischemic but viable myocardium. Subjects that can significantly benefit from the therapeutic vasculogenesis according to the methods of the invention are those who have large areas of viable myocardium jeopardized by the impaired perfusion supplied by vessels that are poor targets for revascularization techniques. Other subjects that can benefit from the therapeutic vasculogenesis methods are those having vessels of small caliber, severe diffuse atherosclerotic disease, and prior revascularization, in particular bypass grafting. Therefore, the therapeutic vasculogenesis according to the methods of the invention can particularly benefit subjects with chronic myocardial ischemia.

Although the compositions may be injected directly into the area of ischemia, the cells are preferably infused into a coronary artery, preferably a coronary artery supplying the area of myocardial ischemia. Where the subject has a totally occluded vessel that would normally supply the area of the ischemic myocardium, the selected coronary artery for infusion is preferably an epicardial vessel that provides collateral flow to the ischemic myocardium in the distribution of the totally occluded vessel.

In one embodiment, the therapeutically effective amount of EGCs is a maximum number of cells that is safely received by the subject. Because the preferred injection route is intracoronary in the case of cardiac ischemia, and cells in culture may become larger than those originally isolated, the maximum dose should take into consideration the size of the vessels into which the cells are infused, so that the vessels do not become congested or plugged. The minimum number of cells necessary for induction of new blood vessel formation in the ischemic myocardium can be determined empirically, without undue experimentation, by dose escalation studies. For example, such a dose escalation could begin with approximately 10⁴/kg body weight of EGCs alone, or in combination with approximately 10⁴/kg CD133⁺ cells. Effective amounts of EGCs sufficient to cause the desired neovascularization can be done based on animal data using routine computational methods. In one embodiment the effective amount is about 1.5x10⁵ EGCs per kg body mass to about 3x10⁵ per kg body mass. In another embodiment the effective amount is about 3x10⁵ per kg body mass to about 4.5x10⁵ EGCs per kg body mass. In another embodiment the effective amount is about 4.5x10⁵ per kg body mass to about 5.5x10⁵ EGCs per kg body mass. In another embodiment the effective amount is about 5.5x10⁵ per kg body mass to about 7x10⁵ EGCs per kg body mass. In another embodiment the effective amount is about 7x10⁵ per kg body mass to about 1x10⁶ EGCs per kg body mass. In another embodiment the effective amount is about 1x10⁶ per kg body mass to about 1.5x10⁶ EGCs per kg body mass. In one embodiment the effective amount of human EGCs is between about 1.5x10⁶ and 4.5x10⁶ EGCs per kg of the subject's body mass and In a preferred embodiment the effective amount is about 5x10⁵ EGCs per kg of the subject's body mass.

In some embodiments of the methods described herein, the composition comprising the EGCs is introduced into a vessel of the subject without substantially altering the arterial pressure. In other embodiments, the composition is introduced into a vessel by blocking arterial flow for an amount of time, such as from five seconds to two minutes, such that the injected cells can pool and adhere to the vessel. In one embodiment, a balloon catheter is used to allow pressure driven administration.

In one preferred embodiment of the methods for inducing neovascularization, the subject is a human. In another embodiment, the subject is an adult, a newborn, an embryo or a fetus. In one embodiment of the methods described herein for treatment of a subject, the cells used in the therapies are isolated from the subject's own umbilical cord blood. Such umbilical cord blood may be cryopreserved at the time the subject is bom for use when needed. In one embodiment of the methods described herein, the treatment of the ischemic tissue, such as but not limited to ischemic myocardium, induces formation of blood vessels supplying blood to the ischemic tissue, blood flow to the ischemic tissue, oxygen supply to the ischemic tissue, or a combination thereof.

### VI. Methods of Providing Cellular Therapies

One aspect of the invention provides methods of providing cellular therapies. One aspect provides a method of providing cellular therapies comprising: (a) purifying one or more cell populations from umbilical cord blood; (b) formulating a cryopreserved preparation including one or more of the cell populations; (c) providing a kit comprising the cryopreserved preparation to a medical care provider.

In a preferred embodiment, one of the cell populations is selected from the group consisting of CD133⁺ cells, EGCs, and mesenchymal stem cells. In another embodiment, one cell population consists of EGCs. In some embodiments, the kit comprises cells isolated from a single umbilical cord, whereas in other embodiments the kit comprises cells isolated from two or more umbilical cords. When the kit comprises cells from a plurality of cord blood samples, the cells may be kept as a single composition or as separate compositions within the kit. When multiple compositions are provided, they may be intended for administration to a single subject or to multiple subjects. Some embodiments of the ongoing methods further comprise isolating one or more cell populations from peripheral blood or from bone marrow for inclusion in the kit or in the composition.

In another embodiment, the kit comprises a buffer for thawing, rinsing or resuspending the cells. The kit may also contain multiple buffers. In another embodiment, the kit comprises additional components, such as angiogenic, antiinflammatory or anticoagulant polypeptides or compounds. In one embodiment, the cellular therapy is ischemia or arterial thrombosis. Other types of ischemia include cardiac, renal, peripheral vascular or cerebral ischemia.

A related aspect of the invention provides a method for distributing any of the EGC compositions described herein, for use by health care professionals, the method comprising placing the composition into a package under sterile conditions and distributing the package for use by health care professionals.

The practice of the present invention will employ, where appropriate and unless otherwise indicated, conventional techniques of cell biology, cell culture, molecular biology, transgenic biology, microbiology, virology, recombinant DNA, and immunology, which are within the skill of the art. Such techniques are described in the literature. See, for example, Molecular Cloning: A Laboratory Manual, 3rd Ed., ed. by Sambrook and Russell (Cold Spring Harbor Laboratory Press: 2001); the treatise, Methods In Enzymology (Academic Press, Inc., N.Y.); Using Antibodies, Second Edition by Harlow and Lane, Cold Spring Harbor Press, New York, 1999; Current Protocols in Cell Biology, ed. by Bonifacino, Dasso, Lippincott-Schwartz, Harford, and Yamada, John Wiley and Sons, Inc., New York, 1999.

The contents of any patents, patent applications, patent publications, or scientific articles referenced anywhere in this application are herein incorporated in their entirety.

### Exemplification

The invention now being generally described, it will be more readily understood by reference to the following examples, which are included merely for purposes of illustration of certain aspects and embodiments of the present invention, and are not intended to limit the invention, as one skilled in the art would recognize from the teachings hereinabove and the following examples, that other stem cell sources and selection methods, other culture media and culture methods, other dosage and treatment schedules, and other animals and/or humans, all without limitation, can be employed, without departing from the scope of the invention as claimed.

### Example 1: Phenotyping EGCs

Characterization was performed by surface phenotyping of UCB unselected mononuclear cells (MNC) and EGCs. UCB-derived EGCs were isolated by adherence of CD133⁻ cells remaining after CD133⁺ selection for 16 hours on fibronectin in EGM2 media (Clonetics). Surface phenotyping was evaluated by incubation for 20 minutes at 4°C with fluorochrome-conjugated mAbs and appropriate isotype controls. An LSR flow cytometer (Coulter, Miami, FL) was used to acquire >5000 fluorescence events per sample. No gating was applied as the entire population of cells were injected into the study animals.

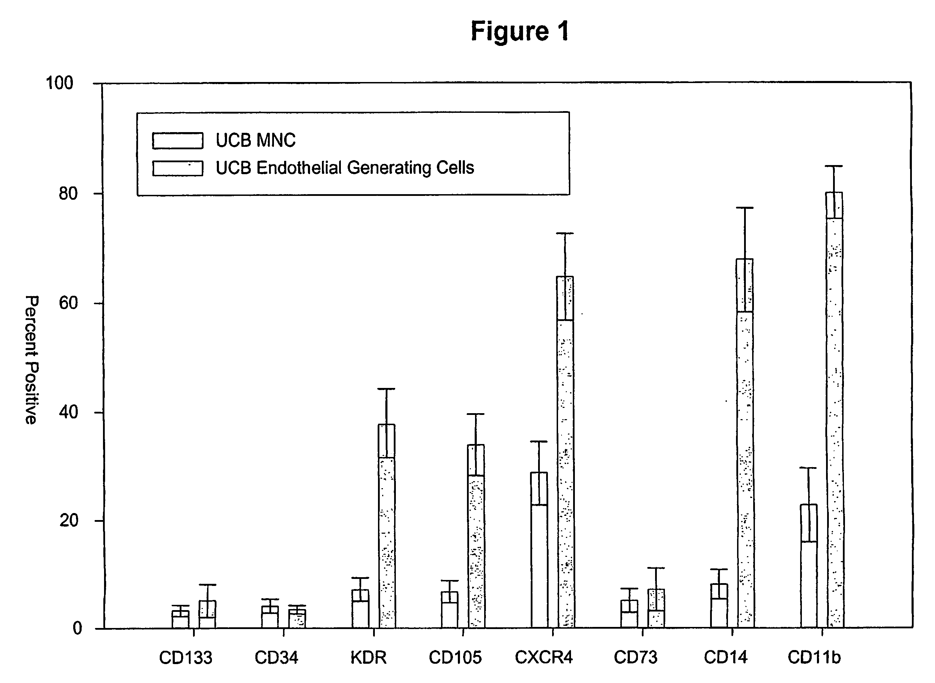

As shown in Figure 1, surface expression in the UCB-derived EGC population was increased for KDR (VEGFR2) 37.6 ⁺/- 6.4%, CXCR4 64.6 ⁺/- 7.8% and CD105 33.8 ⁺/- 5.8% surface expression, compared to the MNC (7.4⁺/- 2.1% KDR, 28.5 ⁺/- 5.8% CXCR4 and 6.7 ⁺/- 2.1% CD105 expression). Also, the surface expression of CD14 was used to assess the proportion of cells expressing this monocyte marker. CD14 expression was noted on 8% of MNC compared to 68% of EGCs.

In addition, UCB-derived EGCs were notable for high expression of CD105 (endoglin), a surface marker for stromal cells, compared to MNC. Higher expression of KDR (VEGFR2) and CXCR4 in the EGC cell population may play a role in homing in response to VEGF and SDF-1 levels *in vivo.*

### Example 2: Ischemic Therapy with EPCs

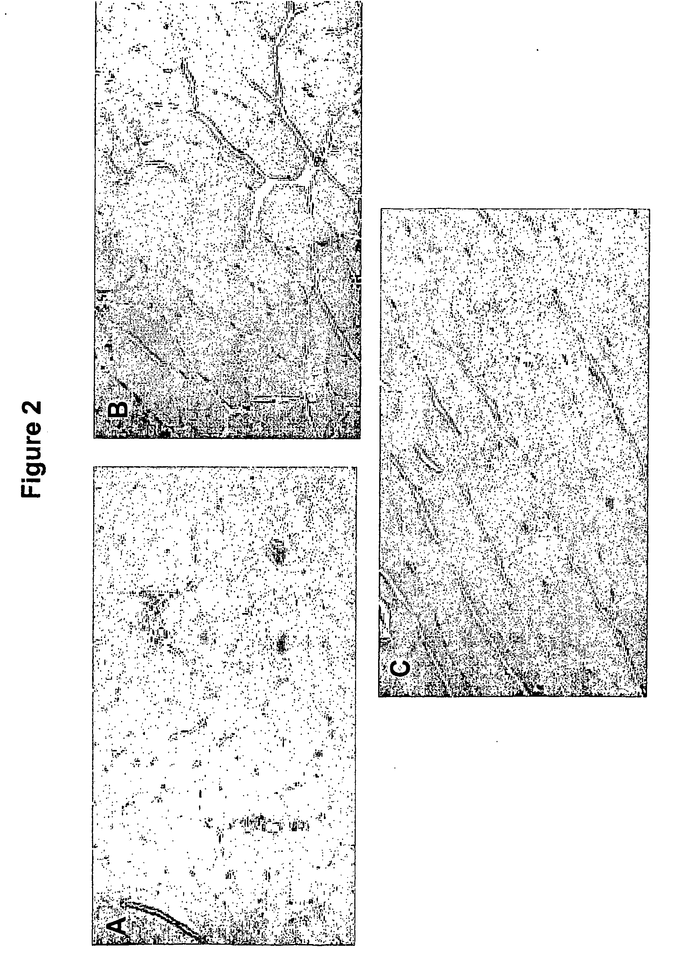

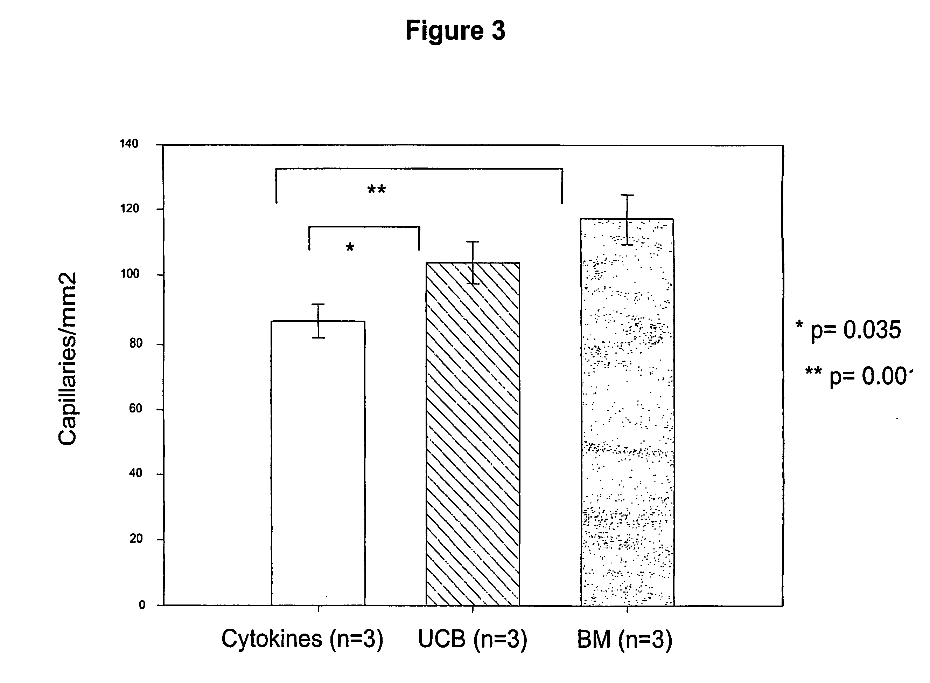

EPCs were isolated as described in U.S. Patent Publication No. 2005/0069527. At 28 days after isolation and ligation of the right femoral artery of SCID/NOD and injection of EPC cells, cytokines or saline, additional tissue was harvested from lower calf muscle of mice and fresh frozen. These specimens were stained for alkaline phosphatase by the indoxyl-tetrazolium method and used to assess capillary density. Capillary density was used to evaluate neovascularization in this murine hind-limb ischemia model. Two blinded investigators counted twenty fields per sample and capillary density was expressed as capillaries/mm². Figure 2 shows a representative slide from each treatment group. In the animals treated with EPCs from UCB the capillary density was significantly increased, 103 ± 6 capillaries/mm² compared to 87 ± 5 capillaries/mm² in control animals (p = 0.035) (Figure 3). Animals that received BM-derived EPCs also showed increased capillary density from control mice (117 ± 8 capillaries/ mm², p= 0.001).

### Example 3: Ischemic Therapy with CD133⁺ Cells and CD34⁻CD133⁻CD73⁻ Cells

We have tested the efficacy of CD133⁺ cells and EGCs in the hind limb vascular injury SCID.NOD mouse model, to determine whether these cells augment neovasculogenesis compared with those animals receiving non-selected MNC alone. This model system has been described in copending U.S. Application No. 10/875643, the contents of which are incorporated by reference herein in their entirety.

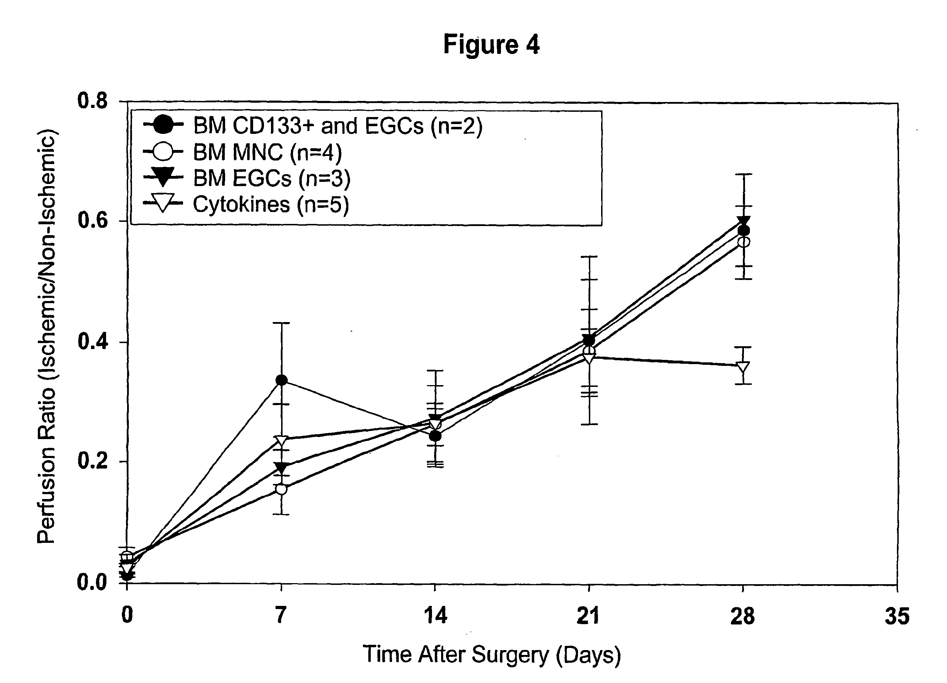

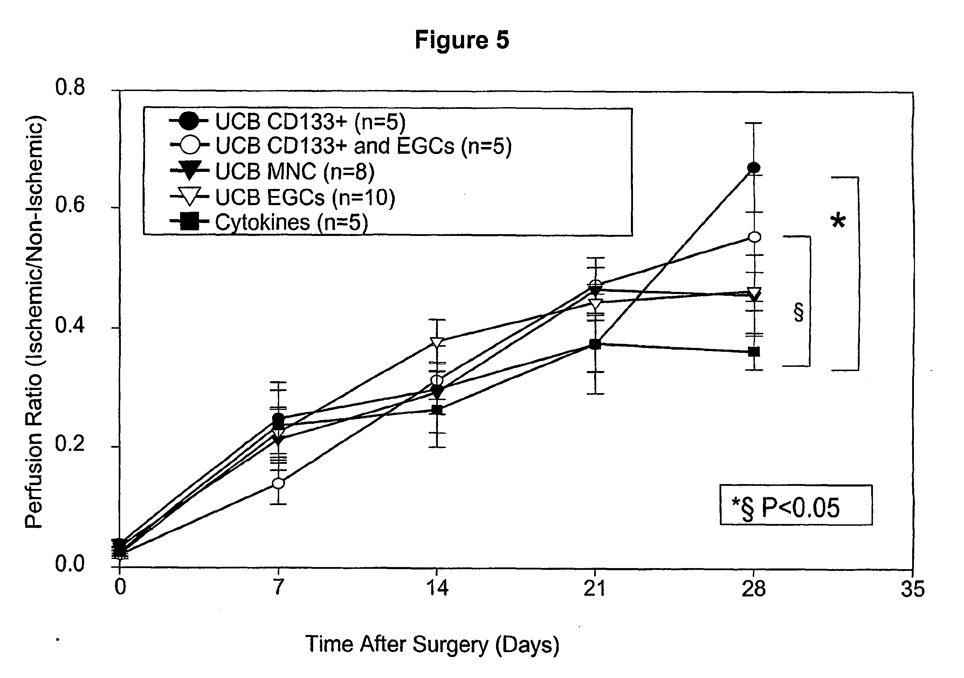

CD133⁺ cells were selected as described in Example 2. EGCs were isolated by adherence of CD133⁻ cells remaining after CD133 selection, for 16 hours on fibronectin in EGM2 media (Clonetics). Ischemia was induced in 40 NOD/SCID mice by ligation of the right femoral artery as described in Example 2. The animals were randomized into 5 groups: control (cytokines), MNC, CD133⁺, EGCs, or a combination of CD133⁺ cells and EGCs at a 1:2 ratio. Doppler flow measurements were taken on both limbs each week for 4 weeks and the ratio of perfusion in the ischemic/healthy limb was used to compare neovascularization in the study groups. The results from the BM derived study are shown in Figure 4. Figure 5 illustrates the results for the UCB derived cells. Immediately following femoral ligation the perfusion ratios showed reduced perfusion in all groups. After 28 days, all treatment groups showed statistically significant improvement in blood flow when compared to the control mice (p<0.05). When BM was used as the source, perfusion ratios after 28 days were 0.46 ⁺/- 0.07 BM MNC (n=4), 0.61 ⁺/-0.08 (n=10) BM EGCs (n=3), and 0.59 ⁺/- 0.01 BM CD133⁺ & EGCs (n=2). The perfusion ratios for UCB at day 28 were: 0.36 ⁺/- 0.07 (n=5) Cytokines (control), 0.57 ⁺/- 0.06 UCB MNC (n=8), 0.67 ⁺/- 0.17 (n=10) UCB CD34-CD133-CD73-, 0.67⁺/-0.08 UCB CD133⁺ (n=5) and 0.55 ⁺/- 0.11 (n=5) UCB CD133⁺ & CD34-CD133-CD73- (n=5).

No differences were noted in animals receiving marrow-derived CD 133⁺ and CD34⁻CD133⁻CD73⁻ cell subsets, compared with non-selected MNC alone. UCB treated animals, however, demonstrated significantly improved blood flow in those receiving enriched CD133⁺ stem cells alone and in combination with CD34⁻CD133⁻CD73' compared with non-selected MNC alone.

### Example 4: Intracoronary infusion of CD133⁺ cells and EGCs in patients with chronic coronary ischemia.

A patient with chronic coronary ischemia, having an area of documented ischemic but viable myocardium supplied by epicardial vessels that provide collateral flow in the distribution of a chronic totally occluded vessel, is eligible for treatment. The eligible patient must report past experience with class II-IV angina as defined by the Canadian Cardiovascular Society. The patient is screened within 30 days of scheduling of a percutaneous coronary intervention (PCI, *e.g.*, balloon angioplasty, stenting, atherotomy, or rotational atherectomy). Areas of ischemic but viable myocardium are identified by exercise/pharmacologic nuclear stress testing in addition to PET scanning. Echocardiographic evaluation of left ventricular ejection fraction (>45% required for patient eligibility) and regional wall motion is part of the initial screening along with complete history and physical examination including a review of concomitant medications, ECG, and baseline laboratory panels (CBC, basic metabolic profile, coagulation panel and acute myocardial infarction panel). Coronary angiography is evaluated for anatomy favorable for the treatment protocol, *i.e.*, chronic total occlusion of an epicardial artery with distal distribution supplied by well established collaterals with a separate culprit vessel amenable to PCI.

Patients ineligible for stem cell treatment include those having coronary lesions amenable to PCI including brachytherapy, contraindications for PCI, cardiac catheterization, bone marrow aspiration, as well as those having had a myocardial infarction within the previous three months, having documented bleeding diathesis, having a known malignancy involving the hematopoietic/lymphoid system, having baseline ECG abnormalities that would hinder interpretation of baseline ECG for ischemia, having severe co-morbidities including renal failure, or having anticipated unavailability for follow-up visits secondary to psychological or social reasons.

Once coronary anatomy in the eligible patient is determined, the patient is removed from the catheterization laboratory and undergoes bone marrow aspiration under conscious sedation. Approximately 150-250 ml of bone marrow aspirate is removed from the iliac crest. Multiple puncture sites may be needed to obtain the desired volume. This volume of bone marrow aspirate yields approximately 10⁶ MNCs. CD133⁺ cells are isolated from the MNCs by labeling with CD133⁺-conjugated magnetic beads followed by automated sorting through magnetic columns (Automacs, Miltenyi). The selected CD133⁺ cells are then washed in buffer solution and can be stored in a concentrated solution of 5 mL. normal saline. The remaining CD133⁻ cells are cultured on fibronectin coated substrates as described in Example 1 to generate EGCs. Alternatively, the CD133⁺ cells, the EGCs, or both, are isolated from umbilical cord blood.

After the patient is given time to recover from the bone marrow aspiration, a PCI is performed. When the operating interventional cardiologist has determined that the PCI is successful, the patient is observed for approximately 5 minutes for any complications. If none, transplantation of stem cells is completed at the same sitting. The epicardial coronary artery, which is the source of collateral vessels to the viable myocardium formerly supplied by a vessel which is now totally occluded, is identified by fluoroscopy. Equipment is passed through an introducer sheath placed in a peripheral vessel for the index PCI. The target parent vessel is cannulated with an infusion catheter through a guide catheter that is placed in the ostium of the appropriate coronary artery in the sinus of Valsalva. Approximately 1 x 10⁴ to 1 x 10⁵ of the isolated cells (CD34⁻CD133⁻CD73⁻ and optionally the CD133⁺ cells) are infused via an infusion catheter into the epicardial vessel supplying the majority of collaterals vessels to the chronically ischemic zone by manual injection of a 5 mL sample over 3 minutes. If the CD133⁺ of cells are also to be administered to the subject, they may be injected at a ratio of 1:1 with the EGCs, although other ratios, ranging from about 1:10 to 10:1, may be used. An additional 2 mL of normal saline is infused through the catheter immediately after the stem cell solution in order to prevent residual cells from accumulating in the catheter proper.

At the end of the cell infusion, a selected coronary angiogram of the vessel is performed to assess TIMI flow and evaluate the integrity of the vessel wall. The patient is monitored in the cardiac catheterization laboratory for 5 minutes post procedure for any complications. The patient is then observed in a monitored setting for 24 hours after the procedure, with an ECG and cardiac enzyme analysis obtained at 8, 16 and 24 hours.

Clinical follow-up evaluation of the patient is performed thereafter at 7, 30, 90, 180 and 365 days. The success of the PCI and stem cell infusion treatment is measured by an improvement in exercise capacity (*e.g*., total exercise duration in seconds, change in exercise duration, time to onset of angina, time to 1 mm ST depression, and the like); major cardiac events (*e.g*., death, revascularization, readmission to hospital secondary to angina, myocardial infarction, and the like); myocardial infarction within 24 hours post procedure or later than 24 hours post procedure (*e.g.*, as measured by an elevation of cardiac enzymes, ECG changes, chest pain not relieved by nitroglycerine, and the like); improvement in anginal symptoms; length of secondary hospital stay; decrease in medication usage; subjective improvement in angina; improvement in left ventricular function and ejection fraction as measured by 2D echocardiogram; improvement in the total area of ischemia compared to initial screening test by nuclear stress testing; and improvement in the viable zone of myocardium compared to initial screening test by PET scan.

### Example 5: Enhancement of Cell Migration towards SDF-1 by AC133⁺ cells and EGCs upon Coculturing

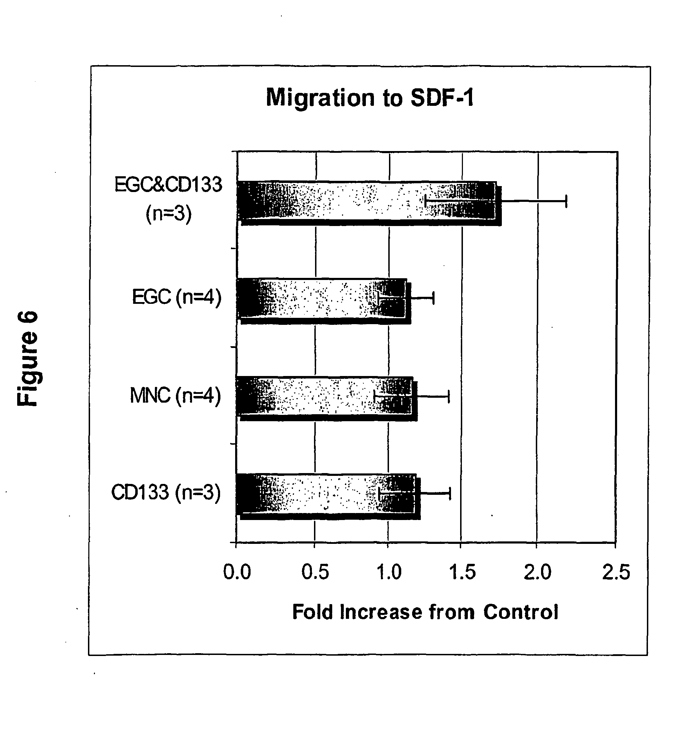

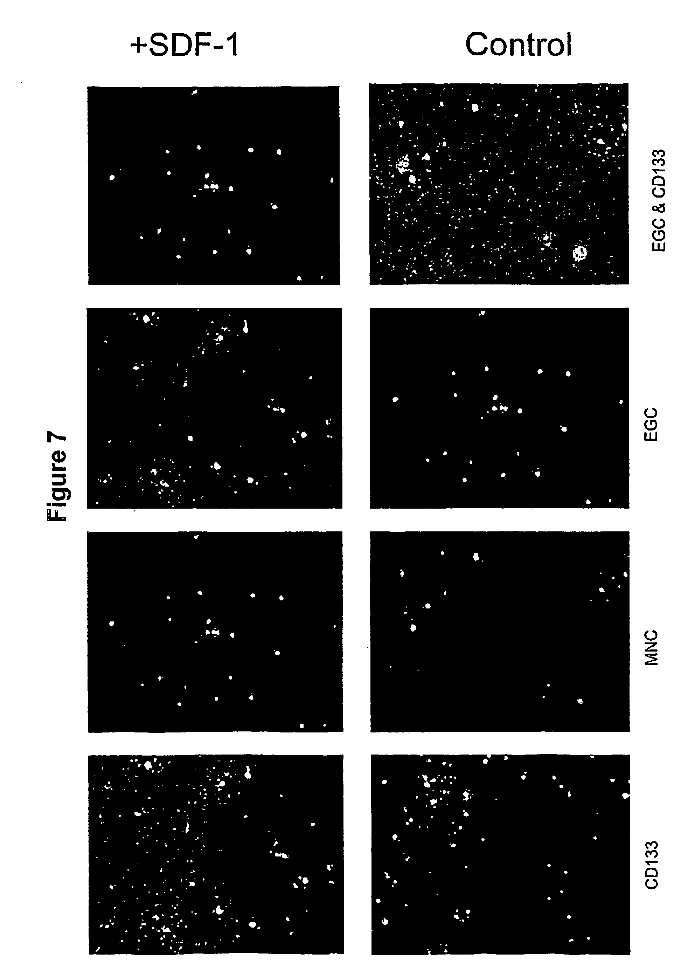

Boyden chamber assays were used to observe chemotactic migration of EGC, CD133, MNC, or EGC & CD133 towards SDF-1. Lower wells ofNeuroprobe 48 well Boyden chambers will be loaded with SDF-1 (100ng/ml). Fibronectin coated polycarbonate membrane with 8µm pores was placed on top of the lower wells and the chamber was assembled. In order to visualize cells, suspensions were pre-labeled with DAPI to stain the nucleus. Cells were pelleted and washed thoroughly before they suspension in either serum-free media or medium with SDF-1. Individual cell suspensions were loaded in the upper wells. The chambers will be incubated at 37°C for 4 hours to permit migration of cells from the upper well, through the membrane, into SDF-1 in the lower wells. Following the 4 hour incubation, the chambers were disassembled and the membrane was removed. Cells were scraped from the upper surface of the membrane leaving only cells that had migrated through the pores in the membrane. Cells were fixed in formaldehyde and mounted on slides. Slides were scanned and quantified by direct cell count of three fields/well and three wells/condition using the 10x objective of an Olympus 480E microscope. Data was expressed as fold increase from the control wells containing media without supplementation (Figures 6 and 7). Our results showed increased migration of EGC & CD133 toward SDF-1 (1.5 fold from control).

### Example 6: Enhancement of HUVEC Tubule Formation by AC133⁺ and EGCs

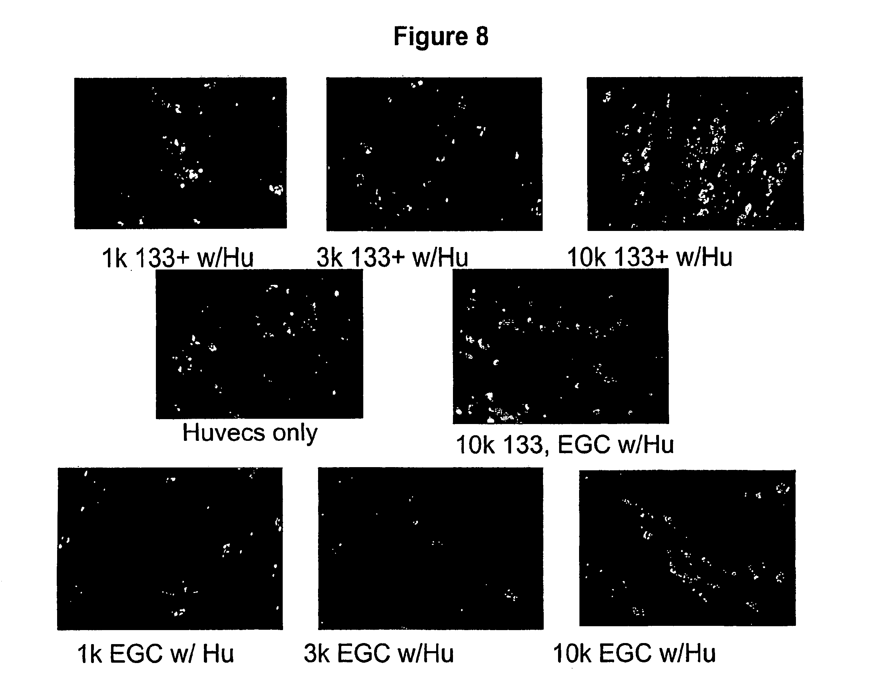

To assess the ability of cord blood-derived endothelial precursors cells (EPC) or CD133⁺ cells to influence tubule formation by committed human umbilical vein endothelial cells, human cardiac fibroblasts were grown to confluency prior to the addition of CM-Dil labeled (fluorescent lypophilic dye) HUVECs in the absence or presence of EPC or CD133⁺ cells. EGCs AC133⁺ cells and EGCs were isolated as describes above. Cell were cultured under hypoxic conditions (5% CO2) and monitored after 24 hours. Tubule formation by HUVECs was measured in the presence of increasing concentrations of AC133⁺ cells, EGCs, or both (Figure 8). Both EGCs and AC133⁺ cells were found to enhance tubule formation in a dosage dependent manner, with maximum tubule formation observed in the presence of both cell types.

## Claims

1. A method of generating a composition of human CD34⁻CD133⁻CD73⁻ cells, the method comprising the steps of
(i) contacting cells from a sample of umbilical cord blood or bone marrow or peripheral blood with a reagent that specifically binds CD133, CD34 or CD73;
(ii) removing, from the sample, cells that bind to the reagent to generate an enriched sample of CD133⁻CD34⁻CD73⁻ cells;
(iii) culturing the enriched sample on a substrate; and
(iv) selecting adherent cells.

2. The method of claim 1, wherein the substrate is a tissue culture container.

3. The method of claim 1 or 2, wherein the cells are cultured for about 4 to 24 hours.

4. The method of any one of claims 1 to 3, wherein the cells are cultured in cell culture medium comprising between 1 and 20% serum.

5. The method of claim 4, wherein the cell culture medium comprises hEGF, hydrocortisone, human albumin, autologous plasma, VEGF, hFGF-B, heparin, IGF-1 and ascorbic acid.

6. The method of any one of claims 1 to 5, further comprising the step of cryopreserving the adherent cells.

7. The method of any one of claims 1 to 6, wherein the CD34⁻CD133⁻CD73⁻ cells express one or more of the surface antigens: CD14, CD11b and CXCR4.

## Patentansprüche

1. Verfahren zum Herstellen einer Zusammensetzung aus menschlichen CD34⁻CD133⁻CD73⁻-Zellen, umfassend die Schritte
(i) Inkontaktbringen von Zellen aus einer Probe von Nabelschnurblut oder Knochenmark oder peripherem Blut mit einem Reagens, das spezifisch CD133, CD34 oder CD73 bindet;
(ii) Entfernen von Zellen, die an das Reagens binden, aus der Probe, um eine angereicherte Probe von CD133⁻CD34⁻CD73⁻-Zellen herzustellen;
(iii) Züchten der angereicherten Probe auf einem Substrat; und
(iv) Selektieren adhärenter Zellen.

2. Verfahren nach Anspruch 1, wobei das Substrat ein Zellkulturgefäß ist.

3. Verfahren nach Anspruch 1 oder 2, wobei die Zellen für etwa 4 bis 24 Stunden gezüchtet werden.

4. Verfahren nach einem der Ansprüche 1 bis 3, wobei die Zellen in Zellkulturmedium gezüchtet werden, das zwischen 1 und 20% Serum enthält.

5. Verfahren nach Anspruch 4, wobei das Zellkulturmedium hEGF, Hydrocortison, menschliches Albumin, autologes Plasma, VEGF, hFGF-B, Heparin, IGF-1 und Ascorbinsäure umfasst.

6. Verfahren nach einem der Ansprüche 1 bis 5, weiter umfassend das Kryokonservieren der adhärenten Zellen.

7. Verfahren nach einem der Ansprüche 1 bis 6, wobei die CD34⁻CD133⁻CD73⁻-Zellen eines oder mehrere der Oberflächenantigen CD14, CD11b und CXCR4 exprimieren.

## Revendications

1. Procédé permettant de générer une composition de cellules CD34⁻CD133⁻CD73⁻humaines, le procédé comprenant les étapes consistant à :
(i) mettre en contact les cellules provenant d'un échantillon de sang du cordon ombilical ou de moelle osseuse ou de sang périphérique avec un réactif qui lie spécifiquement CD133, CD34 ou CD73 ;
(ii) enlever de l'échantillon les cellules qui se lient au réactif pour générer un échantillon enrichi en cellules CD133⁻CD34⁻CD73⁻ ;
(iii) cultiver l'échantillon enrichi sur un substrat ; et
(iv) sélectionner les cellules adhérentes.

2. Procédé selon la revendication 1, dans lequel le substrat est un récipient de culture tissulaire.

3. Procédé selon la revendication 1 ou 2, dans lequel les cellules sont cultivées pendant environ 4 à 24 heures.

4. Procédé selon l'une quelconque des revendications 1 à 3, dans lequel les cellules sont cultivées dans un milieu de culture cellulaire comprenant entre 1 et 20 % de sérum.

5. Procédé selon la revendication 4, dans lequel le milieu de culture cellulaire comprend du hEGF, de l'hydrocortisone, de l'albumine humaine, du plasma autologue, du VEGF, du hFGF-B, de l'héparine, de l'IGF-1 et de l'acide ascorbique.

6. Procédé selon l'une quelconque des revendications 1 à 5, comprenant en outre l'étape consistant à cryoconserver les cellules adhérentes.

7. Procédé selon l'une quelconque des revendications 1 à 6, dans lequel les cellules CD34⁻CD133⁻CD73⁻ expriment un ou plusieurs des antigènes de surface : CD14, CD11b et CXCR4.
